# EUROPEAN PATENT SPECIFICATION

(11) **EP 1 941 914 B1**
(45) Date of publication and mention of the grant of the patent: **26.08.2009**
(21) Application number: 07024809.1
(22) Date of filing: 20.12.2007
(51) Int. Cl.: A61L 2/18, A61L 2/24, A61B 19/00, B08B 3/00, B08B 3/04, G05D 7/06

(54) **Endoscope washer disinfector and method of controlling the supply of the cleaning and disinfecting agent used thereby**
Gerät zum Spülen und Desinfizieren von Endoskopen und Verfahren zur Steuerung der Zuführung des dabei verwendeten Spül- und Desinfiziermittels
Machine à laver et à désinfecter les endoscopes et procédé pour contrôler l'administration de l'agent de nettoyage et de désinfection utlilizé dans ladite machine

(30) Priority: 20.12.2006 JP 2006343480
(43) Date of publication of application: 09.07.2008
(73) Proprietor: OLYMPUS MEDICAL SYSTEMS CORP., Shibuya-ku Tokyo 151-0072 (JP)
(72) Inventor: Kawachi, Shinichiro, Shibuya-ku Tokyo 151-0072 (JP); Suzuki, Eiri, Shibuya-ku Tokyo 151-0072 (JP); Suzuki, Shintaro, Shibuya-ku Tokyo 151-0072 (JP)
(74) Representative: von Hellfeld, Axel

(56) References cited:
- US-A- 3 797 744
- US-A- 4 526 623
- US-A- 5 755 894

## Description

### CROSS REFERENCE TO RELATED APPLICATION

The present application relates to and incorporates by reference Japanese Patent application No. 2006-343480 filed on Dec. 20, 2006.

### Background of the Invention

### (The field of the Invention)

The present invention relates to an endoscope washer disinfector that washes and disinfects used endoscopes, and in particular, to an endoscope washer disinfector that supplies liquid agents necessary for the washing and disinfection and a method of controlling the supply of the agents used by the endoscope washer disinfector.

### (Related art)

A medical endoscope has an elongated, thin and flexible insertion tube. By inserting the insertion tube into body cavities of a patient, it is possible to visually observe internal organs and performs various treatments using therapeutic Instruments. Such therapeutic instruments are inserted into endoscopic ducts (called "channels) formed through the insertion tube. Once the insertion tube is inserted into a patient's body cavity, the surfaces of the insertion tube are subjected to adherence of contamination from body fluids such as mucous membrane, blood, and waste materials. Thus it is always necessary to sufficiently wash and disinfect used endoscopes.

The apparatuses for washing and disinfecting used endoscopes include various types of apparatuses. For example, Japanese Patent Application (Laid-open) Publication No. 1-126947 discloses an endoscope washer disinfector which performs washing, disinfection, and rinsing processes (steps). In each process, a liquid agent is pressurized by a feed pump via an electromagnetic valve, before the pressurized agent is guided to a water supply duct and by injected into a washing bath from an injection nozzle. In this endoscope washer disinfector, a flow sensor senses the amount of liquid agent to be injected automatically and, using the sensed amount of liquid, a predetermined amount of agent is injected into a feed tank.

On the other hand, this kind of endoscope washer disinfectors may include a pressurizing type of supply mechanism. This mechanism provides tanks with air whose pressure is higher than the atmosphere, in which the tanks contain liquid agents such as a washing agent and an disinfecting agent. Thus, a difference in pressure from the atmosphere can be utilized in the tanks, resulting in that the agents within the tanks are pushed out to the washing bath.

In this pressurizing type of endoscope washer disinfector, shut-off means, such as an electromagnetic valve, is disposed at a position along an air-supply-side duct so as to control the amount of each liquid agent to be supplied from each tank. In order to gain proper washing/disinfection effects, it is required to supply amounts of agents properly preset to each of the washing process, disinfection process, and drying process. In particular, the washing and disinfection agents have to be diluted at predetermined concentrations using dilution water.

However, in this type of washer disinfector, even when the shut-off means stops feeding the air, the agent continues to flow along an output-side duct connected with the washing bath until the pressure within the tank realizes the equilibrium with the atmosphere. Hence it is difficult to accurately control the supply of the agents.

In addition, this pressurizing type of endoscope washer disinfector is provided with a flow monitor disposed at a given position in the supply duct. The flow monitor is provided with a bladed wheel to monitor the amount of a liquid agent to be supplied, on the basis of the number of rotations of the bladed wheel. When a shortage of the amount of an agent supplied is detected on information detected by the flow monitor, an error message is issued for a warning. For such an error message, the user has to refill the tank with the new agent. Whenever the pressure in the tank is higher than the atmospheric pressure, the operator has to reduce the pressure in the tank down to the atmospheric pressure, before starting the refilling work. However, when an operator opens the tank without previously reducing the pressure in the tank, there is a fear that the agent which is still present in the tank splashes outside the tank. Thus, the user may be contaminated with the splashing agent.

Moreover, when refilling the tank with the new agent, it is unavoidable that the air is mixed into the supply duct. With this air mixed in, the amount of an agent to be supplied is detected. In such a case, the agent supplied results in being less than a specified volume. To avoid this drawback, the conventional endoscope washer disinfector supplies an agent whose amount is lager than the present value by a predetermined margin. Even when such a manner is adopted, it is not always true that the amount of the margin agrees with an actual amount of air mixed into the supply duct. Thus, the amount of the agent supplied may be poor in accuracy.

US 5,755,894 relates to an endoscopic instrument cleaning apparatus including a syringe coupled to a flushing chamber by a form-fitting stopper. A distal, or instrument receiving end of the flushing chamber is inserted into a supply of cleaning fluid and the plunger of the syringe is drawn out to its fully retracted length, thereby filling the syringe and the flushing chamber with cleaning fluid. Then the distal end of an endoscopic instrument is inserted about through an aperture in a stopper having the shape of a conical frustum and is inserted into the flushing chamber to a depth of about 10 cm and the plunger of the syringe is thrust forward to inject pressurized cleaning solution through the endoscopic instrument. Alternatively, the syringe receiving stopper is recessed within a proximal end of the flush chamber by about 0,95-1,00 cm and a tightly fitting tubular insert is glued into the recess to eliminate the possibility that the syringe receiving stopper will be blown out of the flushing chamber by fluid pressure when it is used. Alternatively, an inward projecting lip is formed in the distal end of the flush chamber for the same purpose. A rectangular flushing board includes a neck having a pair of aligned yokes for seating and holding the syringe body to eliminate wrist and thumb strain. A flushing chamber can be formed as an extension to the body of a syringe, thereby eliminating the need for a separate flushing chamber.

US 4,526,623 refers to a method of cleaning an endoscope, wherein a stop having a communication path is mounted on the open ends of an air/liquid supply valve cylinder and a suction valve cylinder of the endoscope so that liquid may flow between the valve cylinders through the communication path. One end of a suction channel, which opens to the distal end of an insertion section of the endoscope, is connected to a liquid tank through a liquid supply tube. An air supply pump is connected to the tank. The pump is operated in this state and supplies the liquid held in the tank to the one end of the suction channel. The liquid supplied to the one end of the suction channel is discharged from a nozzle at the distal end of the insertion section and the other ends of the suction channel, an air supply channel and a liquid supply channel, which open to a connector mounted on a light guide of the endoscope, through the three channels and the valve cylinders, thereby cleaning the interior of these channels and valve cylinders.

### Summary of the Invention

The present invention has been made due to the above circumstances, and an object of the present invention is to provide a pressurizing type of endoscope washer disinfector and a method of supplying agents, which are able to prevent a liquid agent splashing when the agent is refilled into a tank, and increase the accuracy of amounts of the agent being supplied.

The endoscope washer disinfector according to the present invention comprising: a bath (5) used to wash and disinfect an endoscope accommodated therein; a tank (11) that stores a liquid agent necessary for washing and disinfecting the endoscope accommodated in the bath; a pressuring member (22,19, 17; 22, 19, 39; 22, 17; 22 44) that pressurizes an inside of the tank; a first supply passage (16) that connects the tank and the pressurizing member; a second supply passage (12) that connects the bath and the tank; a discharging member (17, 28, 27; 29, 30; 49, 50) that discharges an inner pressure of the tank to an atmosphere; a detector (13) that detects an amount of the agent extruded from the tank to pass along the second supply passage in response to pressurization of the pressurizing member to the tank and outputs a detection signal indicative of the detected amount of the agent; supply starting means (S1) that causes the tank to start to supply the agent to the bath through the second supply passage by driving the pressuring member to pressuring the inside of the tank through the first supply passage; determining means (S4) that determines whether or not the amount of the agent passing through the second supply passage reaches a specified volume on the basis of the detection signal outputted from the detector; and supply ending means (S5) that ends the supply of the agent to the bath by driving the discharging member to open the inside of the tank to the atmosphere, when the determining means determines that the amount of the agent reaches the specified volume.

The method of supplying a liquid agent from a tank (11) containing a liquid agent for process of an endoscope to a bath (5) in which the endoscope is accommodated and which is for washing and disinfecting the endoscope by pressurizing an inside of the tank, the process including washing, disinfecting, and drying the endoscope, the method comprising steps of: starting (S1) supply of the agent from the tank to the bath by pressurizing the inside of the tank; determining (52, S4) whether or not an amount of the agent being actually supplied to the bath reaches a specified volume on the basis of a detection signal showing the agent pushed outside from the tank in response to the pressurization into the tank; and ending (S5) the supply of the agent to the bath by making an inner pressure of the tank open to an atmosphere, when it is determined that the amount of the agent reaches the specified volume.

Thus, according to the present invention, it is possible to prevent the agents splashing outside, when the agents for washing, disinfecting and/or drying a used endoscope are refilled, while it is possible to accurately supply necessary amounts of the agents.

### Brief Description of the Drawings

In the accompanying drawings:
Fig. 1 is an outlined perspective view showing the outer appearance of an endoscope washer disinfector, whose top cover is opened, according to an embodiment of the present invention;
Fig. 2 is an outlined view showing both a primary duct configuration and a partial electrical configuration in the endoscope washer disinfector;
Figs. 3 - 5 are illustrative views each explaining a partial duct configuration necessary for supplying liquid agents and the flows of the agents to be supplied;
Fig. 6 is an illustrative view explaining a partial duct configuration necessary for supplying the agents in an endoscope washer disinfector according to a first modification of the embodiment;
Fig. 7 is an illustrative view explaining a partial duct configuration necessary for supplying the agents in an endoscope washer disinfector according to a second modification of the embodiment;
Fig. 8 is an illustrative view explaining both a partial duct configuration necessary for supplying the agents and a partial electric configuration in the endoscope washer disinfector;
Fig. 9 is an illustrative view explaining the supply of a predefined amount of agent to a washing bath using a flow monitor;
Fig. 10 exemplifies a flowchart showing the control executed by a controller when the predefined amount of agent is supplied to the washing bath;
Fig. 11 is an illustrative view explaining the supply of the agent to the washing bath;
Fig. 12 an illustrative view explaining the supply of the agent to the washing bath in a state where there is no agent in a tank provided in the endoscope washer disinfector;
Fig. 13 exemplifies another flowchart showing the control executed by a controller when the predefined amount of agent is supplied to the washing bath;
Figs. 14 - 19 are other illustrative views each explaining the supply of the agent to the washing bath;
Fig. 20 is an illustrative view explaining a partial duct configuration necessary for supplying the agents in an endoscope washer disinfector according to a third modification of the embodiment; and
Fig. 21 is another illustrative view explaining the duct configuration of the endoscope washer disinfector according to the embodiment.

### Detailed Description of the Preferred Embodiments

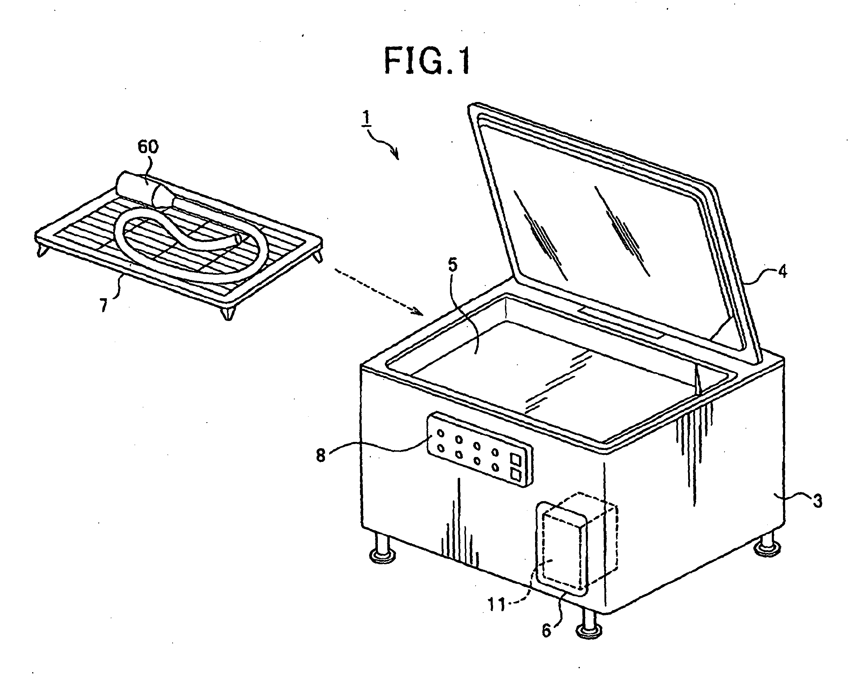

Referring to Figs. 1- 21, an embodiment and its modifications of an endoscope washer disinfector according to the present invention will now be described.

Fig. 1 shows the outer appearance of an endoscope washer disinfector 1 according to the present embodiment. As shown in Fig. 1, the endoscope washer disinfector is provided with a main body 3 and a top cover 4. The main body 3 has an upper part, at which a washing/disinfecting bath 5 (hereinafter, simply referred to as a washing bath) of a predetermined depth is provided. Further, the main body 3 has a front on which an operation panel 8 is provided. Using this operation panel 8, various pieces of information can be given to the washer disinfector and various pieces of output information, such as character information, can be displayed.

The top cover 4 is a cover member formed to have a specified shape and made of hard optical-transparent resin, such as hard transparent resin or hard semi-transparent resin. The top cover 4 is hinged at a predetermined edge portion of the washing bath 5 so that the cover can open/close the opening of the washing bath 5. An operator is thus able to close the opening of the washing bath 5 with the top cover 4, and in this close state, the operator is allowed to visually monitor the inside of the bath 5 through the top cover 4. A support net 7, on which a used endoscope 60 to be washed and disinfected, is placed in this bath 5.

In the front of the main body 3, a small door 3 is provided. The main body 3 has an inner space which can be opened/closed by the door 6. At the inner space, there are provided tanks 11 in which various types of agents, such as washing-agent concentrate solution, disinfecting-agent concentrate solution, and alcohol, are stored. Thus an operator is able to open the door 6 to refill the agents into the tanks.

In the endoscope washer disinfector 1 according to the present embodiment, medical devices such as components and parts of the endoscope 60, therapeutic instruments with openings, and overtubes, can be washed and disinfected. For this washing and disinfection, the dedicated support net 7 is used on which those devices are mounted.

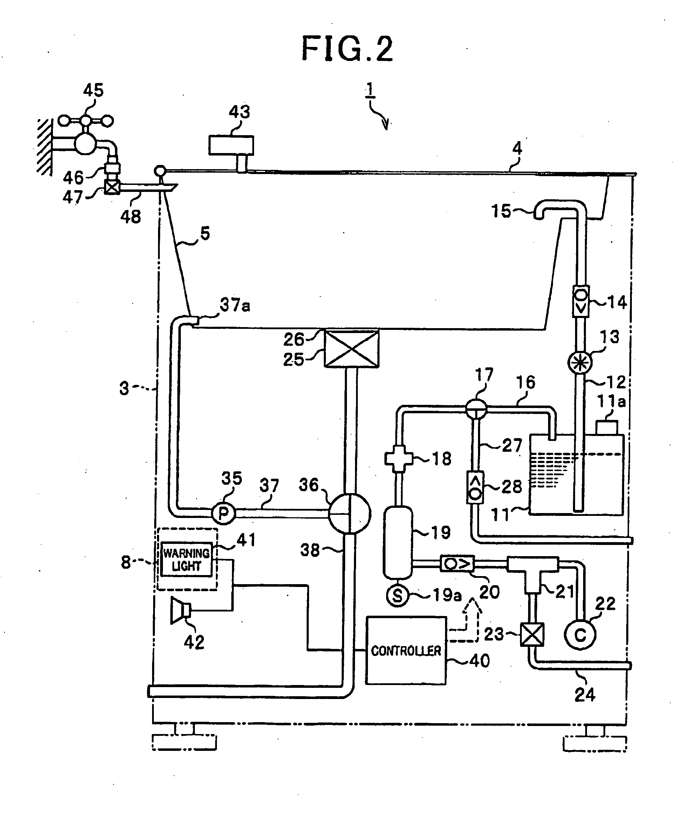

Using Fig. 2, the configuration of this endoscope washer disinfector 1 will now be detailed.

As shown in Fig. 2, the washing bath 5 is connected to a water supply duct 48 through which tap water flows from a tap 45. This water supply duct 48 is provided with a filter 46 and a water supply electromagnetic valve 47. The washing bath 5 has a bottom provided with a drain output 26 to discharge and circulate liquid in the bath 5.

Within the main body 3, various ducts and devices other than the tanks 11 shown in Fig. 1 are arranged. Specifically, within the main body 3, the drain outlet 26 has its secondary part composed of an electromagnetic valve 25 and this valve 25 is connected to one end of an air-discharge duct 38. The air-discharge duct 38 composes a drain which discharges the liquid in the bath 5 from the other end thereof.

At a given position along the path of the air-discharge duct 38, there is installed a three-way electromagnetic valve 36, to which one end of a circulation duct 37 is coupled. At a given position along this circulation duct 37, a circulation pump 35 is installed. The other end of the circulation duct 37 is coupled to the washing bath 5 to provide a circulation outlet 37a at a position on the bottom of the bath 5. That is, within the main body 3, this endoscope washer disinfector 1 circulates the liquid within the bath 5 from the drain outlet 26 to the circulation outlet 37a through the circulation duct 37, so that the used endoscope 60 is subjected to washing and disinfection in the bath 5.

The tank 11, in which the agent (liquid) is stored, is subjected to airtight connection of an agent supply duct 12 which serves as a liquid supply duct. This agent supply duct 12 has both ends, in which one end provides an agent nozzle 15 to supply agent to the washing bath 5 and the other end is located at a position over, but near the bottom of the tank 11. At given positions along this agent supply duct 12, there are disposed a flow monitor 13 serving as a flow sensor and a check valve 14 in this order from the tank side. Though it will be detailed later, the agent supply duct 12 is designed to have a given length and a given inner diameter, which makes it possible that the duct 12 store therein a given amount of liquid agent sufficient for one process of washing/disinfection of the endoscope 60.

The tank 11 is provided with a lid member 11a to close its spout so that the agent (concentrate solution) is prevented from being dried. When refilling the agent, the lid member 11a is opened. In addition, the tank 11 airtightly accepts one end of a gas supply duct 16 to supply gas such as air into the tank, where the one end of the duct 16 is located at a given upper-end position inside the tank.

The other end of this air supply 16 is coupled to a compressor 22 which acts as pressurization means. At given positions along this gas supply duct 16, there are disposed a three-way electromagnetic valve 17 acting as one part of air-discharge means, a speed controller 18, a constant-pressure tank 19, a check valve 20, and a three-way branch duct 21 in this order from the tank side.

Coupled to this three-way electromagnetic valve 17 is one end of an air-discharge duct 27 serving as an exhaust duct. This air-discharge duct 27 has a check valve 28 inserted at a position along the duct 27.

The speed controller 18 is a member to adjust, to a given value, the amount of gas to be supplied from the compressor 22. The constant-pressure tank 19, which is provided with a pressure sensor 19a to sense the pressure therein, is a member to store the gas from the compressor 22 at a specified pressure.

The three-way branch duct 21 is coupled to one end of a relief duct 24 provided with a relief valve 23. Specifically, when the gas is supplied from the compressor 22 to the constant-pressure tank 19 at a given pressure, the relief valve 23 discharges an excessive among of gas to the outside.

In the main body 3, there is also a controller 40 which is control means. This controller 40 receives signals from the operation panel 8. The signals indicate operational commands such as the start and stop of drive of the washer disinfector. In response to such signals, the controller 40 controls the drive of a circulation pump 35, a compressor 22, and electromagnetic valves 17, 25 and 47 on the basis of producers given by programs adjustable. The controller 40 also receives signals detected by the flow monitor 13 and the pressure sensor 19a.

To be specific, the controller 40 is subjected to adjustment of the washing and disinfection programs when washing and disinfection work for the endoscope 60 (refer to Fig. 1) is performed. Based on such programs and the detection signal of the flow monitor 13, the controller 40 controls of the drive of the circulation pump 35, compressor 22, and electromagnetic valves 17, 25 and 47. The controller 40 has also electrical connections with a warning light 41 on the operation panel 8, and an alarm 42 placed at a given position in the main body 3. Thus if a malfunction occurs, the controller 40 will controls the warning light 41 and the alarm 42 to give an alarm.

A deodorization cover 43 is attached to the top cover 4 so that irritant odor from disinfecting solution and others in the washing bath 5 is deodorized.

The endoscope water disinfector 1 of the present invention thus uses various liquid agents such as washing agent, disinfecting agent, and alcohol to perform various processes including a washing process, a disinfection process, and a simplified drying process with the use of alcohol flashing, so that the unused endoscope 60 can be washed and disinfected.

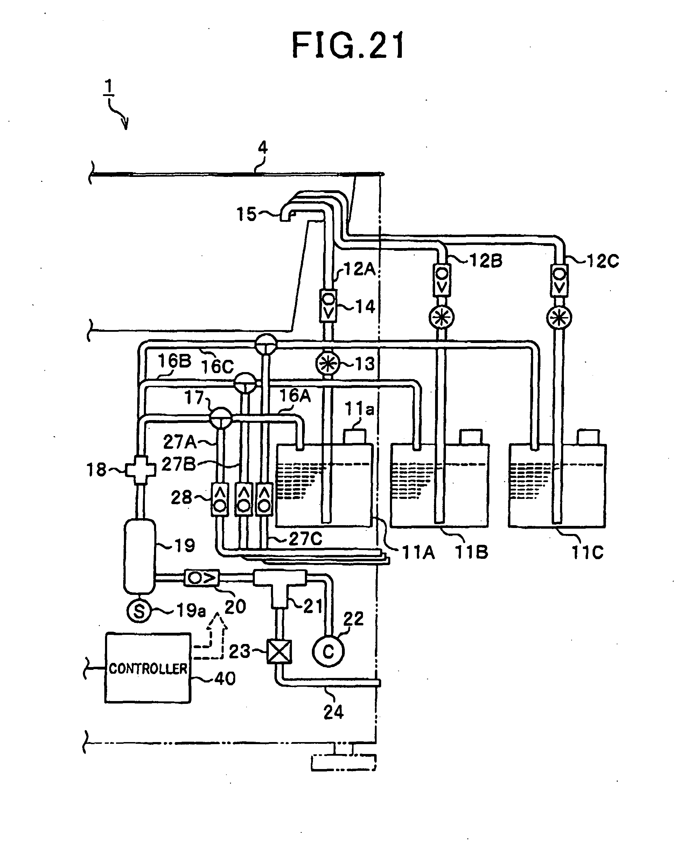

In the present embodiment, for the sake of a simplified explanation, only one duct system among plural duct systems for supplying a plural of types of agents to the washing bath 5 is shown. However, in effect, the foregoing agent supply duct system is provided every type of agent. Actually, as partially shown in Fig. 21, the tanks 11 (11A, 11B and 11C) that respectively store a plurality of types of agents (washing agent, disinfecting agent, and alcohol) are disposed within the main body 3 and duct systems (16A, 16B and 16C, and 12A, 12B and 12C) and various other elements such as switch valves and sensors are disposed in the same way as the foregoing. This makes it possible to use only one agent-supply pressure source provided with one compressor 22 and one constant-pressure tank 19 so that the plural types of agents can be supplied with the same pressure system, thus making the pressure source compact.

In particular, the agent supply duct system according the present embodiment may be applied to systems to supply only the washing agent for the washing process and alcohol for the drying process. The duct inner volume defined by the inner diameter and the length of each duct can be changed depending on the type of an agent to be used.

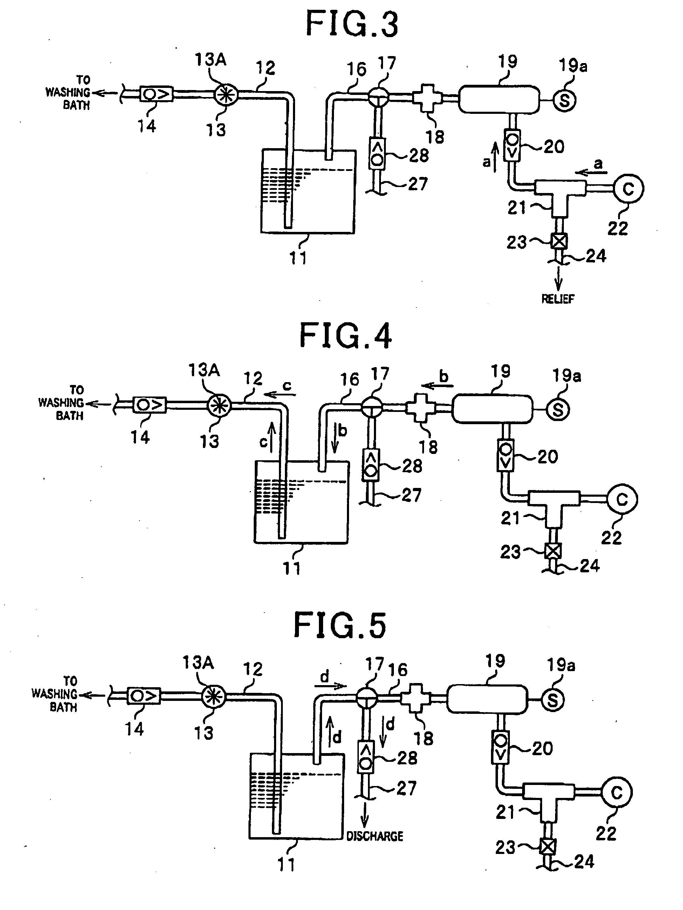

Referring to Figs. 3 - 19, the operations performed by the endoscope washer disinfector 1 according to the present embodiment will now be described. This description focuses operations to supply the agents to the washing bath 5.

At first, referring to Figs. 3 - 5, description will be made to switchovers among the supply passages of each liquid agent which is required to accurately supply the liquid agent to the washing bath 5 by a predetermined amount. These switchovers are executed in response to the commands from the controller 40, of which control example will be described later.

In the initial state, the constant-pressure tank 19 and the gas supply duct 16 have the internal pressure which is the same as the atmospheric pressure. In this state, of input/output three ports of the three-way electromagnetic valve 17, the port which leads to the constant-pressure tank 19 is closed and the port which leads to the tank 11 is opened, so that the valve 17 is switched to communicate with the air-charge duct 27 to realize the atmospheric open state.

When the endoscope washer disinfector performs the washing and disinfection processes of the endoscope 60, the controller 40 shown in Fig. 2 issue a command to supply the gas to the constant-pressure tank 19 in order to supply the agent in the tank 11 to the washing bath 5. That is, the compressor 22, which is started up in advance, is driven to supply the gas along a direction shown by an arrow "a" in Fig. 3. This supply continues until a predetermined pressure of the gas is realized in the tank 19. The controller 40 detects the pressure in the tank 19 using an detection signal from the pressure sensor 19a. When the predetermined pressure in the tank 19 is obtained, the drive of the compressor 22 is stopped.

During the period until this predetermined pressure state, the relief valve 23 works to release an excessive gas through the relief duct 24 to the outside so that the pressure within the constant-pressure tank 19 is kept at the predetermined value.

At the start of the preprogrammed washing and disinfection processes, the controller 40 controls a positional switchover of the ports of the three-way electromagnetic vale 17 such that the constant-pressure tank 19 and the tank 11 communicates with each other, instead of the position communicating with the air-discharge duct 27. This switchover allows the gas in the constant-pressure tank 19 to be fed into the tank 11 through the gas supply duct 16, as shown by an arrow "b" in Fig. 4.

This gas supply will raise the pressure in the tank 11, resulting in that the agent in the tank 11 is pushed into the agent supply duct 12, as illustrated by an arrow "c" in Fig. 4, to be supplied to the washing bath 5.

The amount of the agent that flows along the agent supply duct 12 is monitored by the flow monitor 13. This flow monitor 13 monitors the flow of each agent and outputs a signal showing one count whenever the flow amount reaches a rated amount previously assigned to the flow monitor 13. The output signal from the flow monitor 13 is given to the controller 40. On completion of detection of a predefined amount of the agent on this signal, the controller 40 immediately controls the valve-member positions of the three-way electromagnetic valve 17 so as to open its port connected to the air-discharge duct 27. Thus the pressure in the tank 11 instantaneously becomes the atmospheric pressure (i.e., opens to the atmosphere).

That is, along a direction shown by an arrow "d" in Fig. 5, the gas compressed within the tank 11 instantaneously flows from the tank 11 to the air-discharge duct 27, thus being discharged until the pressure in the tank realizes an equilibrium condition with the atmosphere. In this way, only the predetermined amount of each agent is supplied to the washing bath 5.

The controller 40 then drives the compressor 22 and receives the signal detected by the pressure sensor 19a. When the pressure in the constant-pressure tank 19 becomes the predetermined value again, the controller 40 stops the drive of the compressor 22.

As described, the endoscope washer disinfector 1 according to the present embodiment is able to accurately supply the amount of each agent used in each washing/disinfection process. In particular, this is useful for the washing agent to be used after being diluted at a specified concentration by adding dilution water, because an amount of agent precisely adjusted for each purpose is supplied to the washing bath 5.

In this endoscope washer disinfector 1, the pressure in the tank 11 is higher than the atmospheric pressure only during the period to supply the agent to the washing bath 5. During the periods other than the gas supply period, the pressure in the tank 11 is opened to the atmosphere via the air-discharge duct 27. Thus, it is not required for operators to manually release the gas in the tank 11 to the atmosphere when refilling the tank 11 with each agent. When the tank 11 is erroneously opened in a state where the tank 11 is in high pressure, it is possible to prevent the agent residing in the tank from splashing outside, whereby the exposure to the agent can be prevented.

Further, in the above endoscope washer disinfector 1, the pressure of the constant-pressure tank 19 will not be opened to the atmosphere. Thus it is possible to shorten a time necessary to drive the compressor 22 for the next process again until the pressure of the tank 19 is raised up to the predetermined pressure.

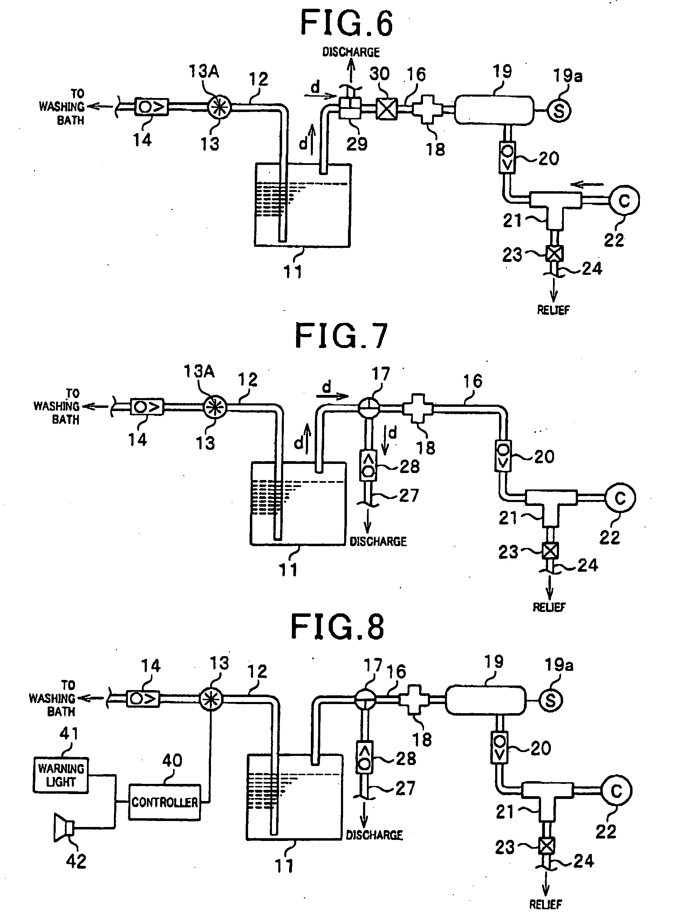

This endoscope water disinfector 1 may be modified as shown In Fig. 6, in which the combination of the gas supply duct 16 with the three-way electromagnetic valve 17 and the air-discharge duct 27 is replaced by another combination. That is, in the gas supply duct 16, there are disposed an air-discharge valve 29 and an open/close type of electromagnetic valve 30 in this order when viewed from the tank 11. The air-discharge valve 29 works to make the inside of the tank 11 open to the atmosphere.

Another modified example of the washer disinfector 1 is shown in Fig. 7, wherein the foregoing constant-pressure tank 19 is removed from the piping configuration shown in Figs. 3 - 5. The present invention is also applicable to such a configuration with no constant-pressure tank 19.

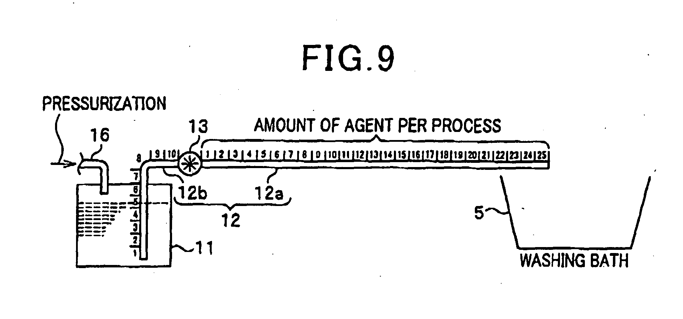

Referring to Figs. 8 and 9, the configurations and operations to measure a predefined amount of the liquid agent and an example of control for refilling each of the agents will now be described.

As described, the controller 40 is able to receive the detection signal from the flow monitor 13 and, if necessary, output signals to the warning light 41 and the alarm 42 for an alarm.

As shown in Fig. 9, the agent supply duct 12 is composed of a first supply duct 12a arranged between the flow monitor 13 and the washing bath 5 and a second supply duct 12b arranged between the flow monitor 13 and the tank 11. The first supply duct 12a has a volumetric capacity which corresponds to an amount of agent (e.g., washing agent, disinfecting agent, or alcohol) necessary for any single (one-time) process among the washing, disinfection, and/or drying processes. Incidentally, in Fig. 9 and subsequent illustrative figures, the check valve 14 is removed form the agent supply duct 12 for simplifying the explanations.

For example, as shown in Fig. 9, the inner volume of the first supply duct 12a is set to a volume corresponding to "25" counts counted by the flow monitor 13. The flow monitor 13 uses a bladed wheel 13A which rotates only when the agent flows therethrough.

That is, in cases where a preset amount of the agent, which corresponds to one of the volumetric capacities obtained by equally dividing the entire volumetric capacity of the first supply duct 12a, is detected, the flow monitor 13 outputs to the controller 40 the signal showing "1" count detected by the rotation of the inner bladed wheel. During this one-count detection, the preset amount of the agent is pushed toward the washing bath 5 from the stored entire agent. When the flow monitor 13 completes the "25" counts, a predefined amount (i.e., an amount of "25" counts) of the washing agent is supplied to the washing bath 5. Meanwhile, the second supply duct 12b has a volumetric capacity which corresponds to "10" counts counted by the flow monitor 13.

In connection with Figs. 9 - 19, an example of the control for supplying the predefined amount of the agent to the washing bath 5 will now be detailed.

At first, when the start switch on the operation panel 8 is switched "on" by an operator, the predetermined process program for washing and disinfecting a used endoscope 60 is started up.

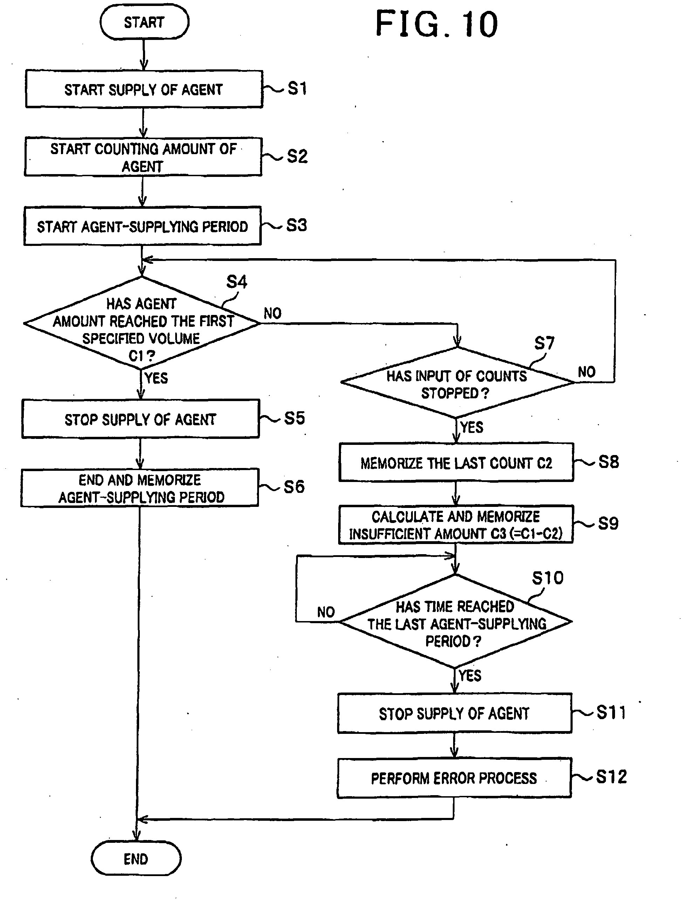

For supplying the washing agent into the washing bath 5 during the performance of the washing and disinfection program, the endoscope washer disinfector 1 activates the processing shown in Fig. 10, where the start of supply of the washing agent is first commanded (step S1). To start the supply of the washing agent, as described, the controller 40 switches the valve member positions of the three-way electromagnetic valve 17 from its position to the air-discharge duct 27 to its position allowing the constant-pressure tank 19 and the tank to communicate with each other. Thus the supply of the washing agent in the tank 11 to the agent supply duct 12 begins.

Next, the controller 40 starts the measurement of the amount of the agent, which is executed using, as measurement unit, a "count" corresponding to one rotation of the bladed wheels of the flow monitor 13 (step S2). Specifically, the flow monitor 13 detects as "counts" the amount of the agent which passes therethrough and output a signal showing the detected counts to the controller 40, so that the controller 40 memorizes the value of the counts. The controller 40 follows step S2 by starting measurement of a time of period which elapses from the start of the agent supply (, which will now be referred to simply as "agent-supplying period") (step S3).

The controller 40 determines, as the next process, whether or not the memorized count reaches a first specified volume C1 (step S4). The first specified volume C1 shows the total amount of the agent which should be supplied to the washing bath 5 for one time of washing or disinfection process. In the present embodiment, "25" counts to be measured by the flow monitor 13 are defined as the first specified volume C1.

At step 54, when the controller 40 determines that the count reaches the first specified volume C1 (i.e., "25" counts are given from the flow monitor 13) yet, the controller 40 controls the three-way electromagnetic valve 17 to open its vale-member position to the air-discharge duct 27 (step S5). Thus the pressure within the tank 11 is instantly brought into a pressure that comes to equilibrium to the atmospheric pressure, whereby the agent supply stops. The controller 40 ends the measurement of the agent-supplying period and memorizes the measured agent-supplying period (step S6), before ending the agent supply to the washing bath 5.

When it is determined at step S4 that the count has not reached the first specified volume C1, the controller 40 shifts to the determination of whether or not the input of the count from the flow monitor 13 is stopped (step 57). If the input of the count still continues, the processing is returned to step S4 again.

When the input of the count from the flow monitor 13 is stopped, the controller 40 memorizes the last count C2, that is, the newest count normally counted (step 58), and calculates an insufficient (running-short) amount of flow of the agent C3 (=C1-C2), with the calculated amount C3 memorized therein (step 59).

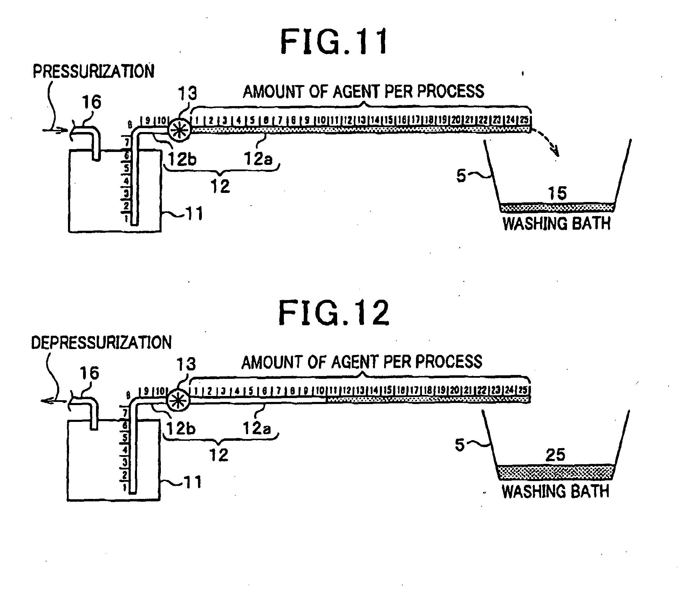

To be specific, in a state where the input of the count from the flow monitor 13 stops at a count of "15", as shown in Fig. 11, this means that the agent has already been supplied to the washing bath 5 by an amount corresponding to "15" counts. This also means that there is no agent within both the tank 11 and the second supply duct 12b. In this state, the bladed wheel of the flow monitor 13 stops from counting the amount of flow of the agent.

The controller 40 memorizes the last count C2 from the flow monitor 13, i.e., in this example, "15" counts, in its internal memory. Next, the controller 40 calculates a difference between the first present value C1 and the last count C2 to produce the insufficient amount C3 (C3=C1-C2) and memorizes its produced amount in the internal memory. In this example, the insufficient amount C3 is "10" counts (10 = 25 - 15).

Step S9 is followed by another step (step S10), where the controller 40 determines whether or not the agent-supplying period starting from the process at step S3 becomes equal to the last agent-supplying period. "The last agent-supplying period" indicates a period of time needed to supply the agent in the last washing or disinfection process of a used endoscope 60. More specifically, the last agent-supplying period is a value stored at step S6 through the last washing or disinfection process. If it is determined at step S10 that the current agent-supplying period becomes equal to the last agent-supplying period, the controller 40 stops the agent supply in the same manner as that at step S5 (step S11).

In the present embodiment, in cases where it is impossible to count any more the flow of the agent by the flow monitor 13, the agent-supplying period is used to control the amount of the agent to the washing bath 5. In this example, as shown in Fig. 11, the control on the last agent-supplying period is performed such that an amount of "10" counts, which is the insufficient amount C3, and an amount of "15" counts, which is the last counted amount already supplied to the washing bath 5, are totaled to be equal to an amount of "25" counts, which is the first specified volume C1.

Controlling the agent supply on the last agent-supplying period is based on the fact that a cubic ratio between the agent and the remaining space both occupied in the tank 11 can well be approximated to a ratio of that in the initial state prior to starting the agent supply. Thus this control is able to minimize an error in supplying the agent.

The controller 40 shifts the processing to step S11, where the agent supply is stopped, the warning light 41 and/or the alarm 42 is driven to operate for error processing, which includes display showing the shortage of the agent in the tank 11 (step S12), before ending the agent supply. Hence an operator can notice that there is a shortage of the agent in the tank 11, and refill the agent the tank 11 with the agent.

Incidentally, in the processes that requiring the supply of the washing agent, disinfecting agent, and alcohol, each agent is diluted at a desired concentration. For this, the dilution water is supplied from the water tap 45 (refer to Fig. 2) into the washing bath 5 via the water filter 46 by controlling the water supply electromagnetic valve 47, before performing the washing, disinfecting, and drying steps in a desired order.

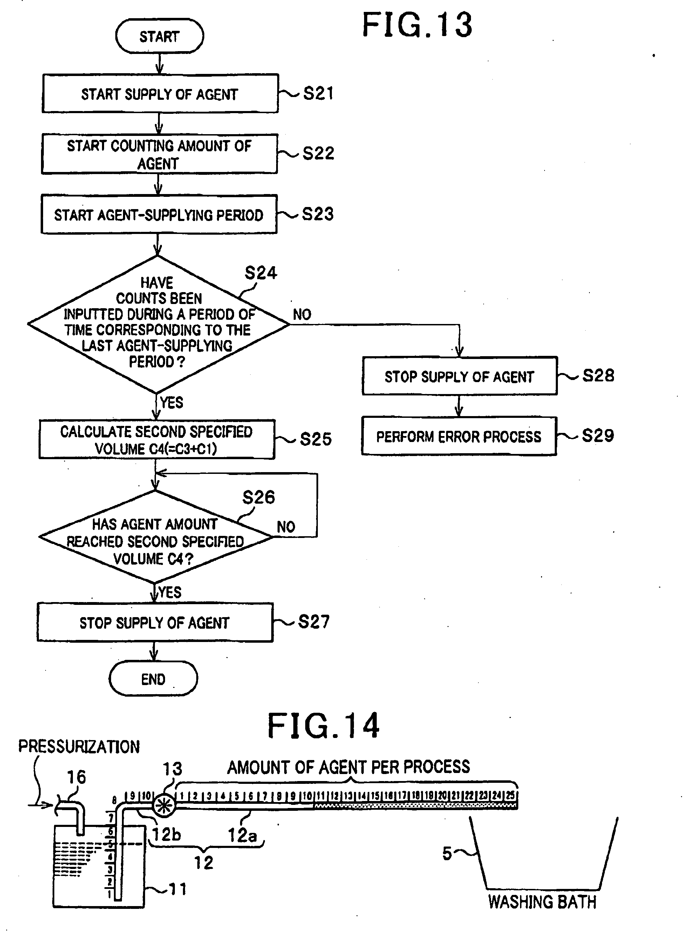

Referring to Figs. 13 - 19, a second control for supplying the agent, which is executed in the endoscope washer disinfector according to the preset embodiment will now be exemplified. This execution example is based on the procedures shown in Fig. 13. This second control follows steps S8 - S12 shown in Fig. 10 according to the first control example, and is directed to the washing and disinfection of a new used endoscope 60.

By way of example, an assumption is made such that, as shown in Fig. 14, an amount of "15" counts of the agent, which was measured using the flow monitor 13, is still left within the first supply duct 12a in the last-time washing or disinfection process.

First, for supplying the agent to the washing bath, the controller 40 commands the start of supply of the agent (step S21). The controller 40 starts to measure the amount of flow of the agent being supplied (step S22), and starts to measure an agent-supplying period, which is an elapse time from the agent supply start (step S23). These processes are the same as those at steps S1 - S3 in Fig. 10 described.

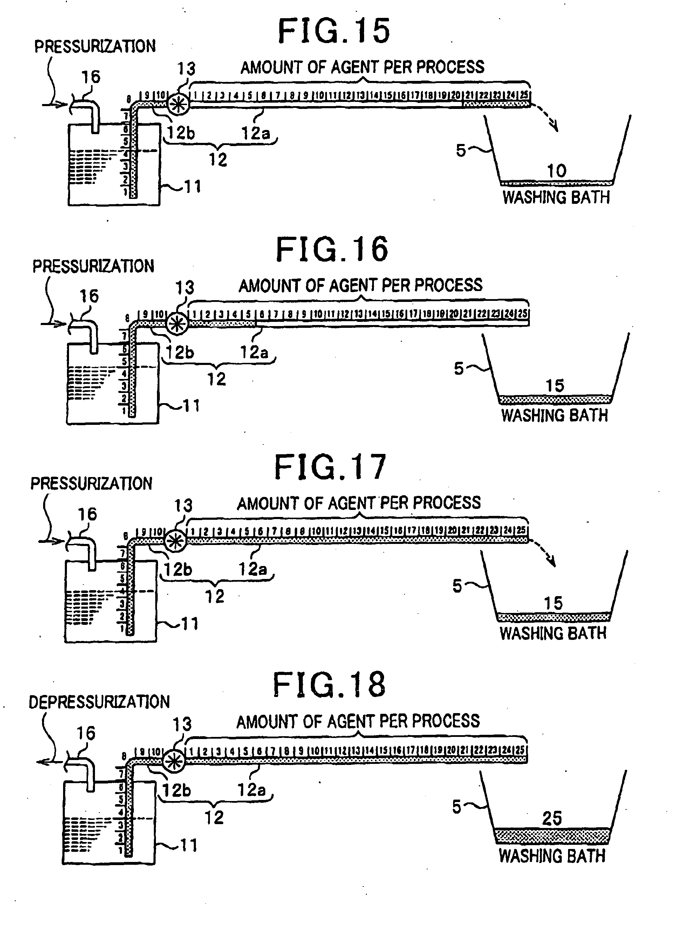

Next, the controller 40 determines whether or not there is an input of counts from the flow monitor 13 within a period of time corresponding to the last agent-supplying period (step S24). In the state shown in Fig. 14 where the agent has not been supplied from the tank 11 into the second supply duct 12b, the bladed wheel of the flow monitor 13 stops from rotating, with no count measured. Additionally, this no-count condition of the flow monitor 13 is kept until the agent already supplied into the second supply duct 12b has not arrived at the flow monitor 13. As shown in Fig. 15, once the agent arrives at the flow monitor 13 via the second supply duct 12b, the force of the agent flow forces the flow monitor 13 to begin counting.

At the time when the counting starts, the amount of a gas (i.e., air contained in the second supply duct) which is equal to the inner volume (corresponding to "10" counts) of the second supply duct 12b has already been pushed out. Hence, as illustrated in Fig. 15, the amount of "10" counts of the agent, which is equal to the inner volume of the second supply duct 12b, has supplied into the washing bath 5. In this state, within the first supply duct 12a, the agent of "5" counts counted by the flow monitor 13 remains.

The controller 40 shifts to step S24, where a second specified volume C4 is calculated in response to the input of counts from the flow monitor 13 (step 525). The calculation of this second specified volume C4 uses the insufficient count C3 memorized when the agent ran short in the last process (refer to step 59 in Fig. 10) in such a manner that addition is made between the insufficient count C3 and the first specified volume C1 (i.e., C4 = C3 + C1). In this example, the insufficient count C3 in the last process is "10" counts and the first specified volume C1 is "25" counts, so hat the second specified volume C4 is "35" counts.

Practically, as illustrated in Fig. 15, when the flow monitor 13 begins its counting, the washing bath 5 has already accepted the agent of "10" counts. Meanwhile, the agent of "5" counts remains within the first supply duct 12a. Additionally, between the agent of "5" counts and the flow monitor 13 in the first supply duct 12a, there is also a layer of air whose volume corresponding to "20" counts, which comes from the compressor 22.

As the agent supply advances, the agent of "5" counts that remained in the first supply duct 12a is supplied into the washing bath 5. Thus, as illustrated in Fig. 16, the washing bath 5 contains an amount of "15" counts of the agent, whilst the flow monitor 13 has counted an amount of "5" counts of the agent, which currently remains in the second supply duct 12b. On the other hand, without newly accepting a fluid of "20" counts, the agent which resides next to the flow monitor 13 in the first supply duct 12a cannot reach the washing bath 5, because there is the layer of gas (air) ahead thereof in the first supply duct 12a.

After this, when the flow monitor 13 has counted an amount of "20" counts of the agent, the agent, which has remained in the first supply duct 12a, finally reaches the washing bath 5, as illustrated in Fig. 17. At the time of this supply, the flow monitor 13 has counted "25" counts of the gas (air) which is equal in amount to the first specified volume C1. After this measurement, the remaining agent of "10" counts counted by the flow monitor 10 is actually supplied to the washing bath 5.

At the next step 526, the controller 40 determines whether or not the count of the flow monitor 13 becomes equal to the second specified volume C4 calculated. The controller 40 proceeds to the next step S27, where, as illustrated in Fig. 18, when the remaining agent of"10" counts is supplied into the washing bath 5, the controller 40 responds to input of the second specified volume C4 of "35" counts from the flow monitor 13 by commanding the stop of the agent supply. This ending process of the agent supply at step S27 is the same as step S5 in Fig. 10.

Meanwhile, it is determined at step S24 that any count is not inputted from the flow monitor 13 during the period corresponding to the last agent-supplying period, the controller 40 decides an error, such as running out the agent in the tank 11 or choking up the ducts. Hence the controller 40 commands that the agent be stopped from being supplied (step S28), and drives the warning light 41 and/or the alarm 42 to perform a process for errors, such as displaying the occurrence of an error (step S29).

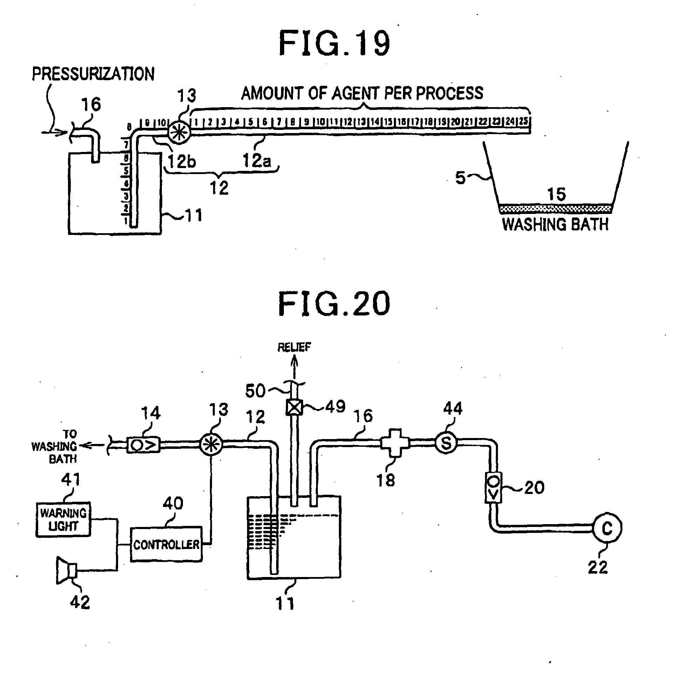

Specifically, when any count is not inputted from the flow monitor 13 during the period corresponding to the last agent-supplying period, the supply state can be illustrated as shown in Fig. 19. That is, there is no agent any more within both the tank 11 and the second supply duct 12b, with no count gained from the flow monitor 13. In this state, all the agent that remained within the first supply duct 12a has pushed forward by the gas supplied from the compressor 22 so as to be fed into the washing bath 5 completely.

Such a complete vacant state occurs, when a user did not refilled the tank 11 with a new agent, even if the error process at step S12 in Fig. 10 has been issued for exchanging the tanks or refilling the agent. Once the error light 41 and/or the alarm 42 issue the error, such error issuing state can be terminated by operator's predetermined operations on the operation panel 8.

As described above, the endoscope washer disinfector 1 is able to control supplying the agent to the washing bath 5 in an accurate manner. In addition, in refilling the agent, it is possible to prevent the agent from splashing outside.

A modification is provided as follows. The foregoing present embodiment employs the constant-pressure tank 19 subjected to pressurization up to a predetermined pressure every initial state thereof. In this configuration, it is required to counterbalance the inner pressure of a pressure bottle 11 to the atmosphere pressure immediately after the end of the agent supply, depending on how much the agent still remains in the pressure bottle 11. In this supply, the speed of the agent to be fed changes depending on the remaining volume of the agent in the bottle 11, even if the pressure given from the constant-pressure tank 19 is constant.

With due consideration of the above drawback, the endoscope washer disinfector 1 takes the duct configuration shown in Fig. 20. As shown, there is no constant-pressure tank, and instead, the gas supply duct 16 is provided with a pressure sensor 44 and the compressor 22 is subjected to the ON/OFF control. In addition, as shown in Fig. 20, a relief duct 50 with a relief valve 49 is directly coupled with the tank 11.

In this duct configuration, the pressure sensor 44 measures pressure to keep a constant pressure in the tank 11. Hence, by driving the relief valve 49 so that the air is discharged from the relief duct 50, it is possible to give a constant flow speed to the agent, whereby the amount of the agent to be supplied can be controlled during the agent-supplying period.

As a result, in the endoscope washer disinfector 1 according to the present embodiment, changes in the flow speed of the agent, which is due to the fact that the bladed wheel of the flow monitor 13 is installed in the agent supply duct 12, it is possible to control the amount of the agent simply and accurately. In addition, in this case, the duct configuration is less expensive in the manufacturing thereof.

Although the description above contains many specificities, these should not be construed as limiting the scope of the invention but as merely providing illustrations of some of the presently preferred embodiments of the present invention. Thus the scope of the present invention should be determined by the appended claims.

## Claims

1. An endoscope washer disinfector comprising:
a bath (5) used to wash and disinfect an endoscope accommodated therein;
a tank (11) that stores a liquid agent necessary for washing and disinfecting the endoscope accommodated in the bath;
a pressuring member (22,19, 17; 22, 19, 39; 22, 17; 22 44) that pressurizes an inside of the tank;
a first supply passage (16) that connects the tank and the pressurizing member;
a second supply passage (12) that connects the bath and the tank;
a discharging member (17, 28, 27; 29, 30; 49, 50) that discharges an inner pressure of the tank to an atmosphere;
a detector (13) that detects an amount of the agent extruded from the tank to pass along the second supply passage in response to pressurization of the pressurizing member to the tank and outputs a detection signal indicative of the detected amount of the agent;
supply starting means (S1) that causes the tank to start to supply the agent to the bath through the second supply passage by driving the pressuring member to pressuring the inside of the tank through the first supply passage;
determining means (54) that determines whether or not the amount of the agent passing through the second supply passage reaches a specified volume on the basis of the detection signal outputted from the detector; and
supply ending means (S5) that ends the supply of the agent to the bath by driving the discharging member to open the inside of the tank to the atmosphere, when the determining means determines that the amount of the agent reaches the specified volume.

2. The endoscope washer disinfector according to claim 1,
wherein the pressurizing member is provided with a single compressor (22) to supply a pressurized gas to the first supply passage.

3. The endoscope washer disinfector according to claim 1,
wherein the pressuring member is provided with a signal compressor (22) to supply a pressurized gas and a single constant-pressure tank (19) to always hold an inner pressure at a given value in accordance with the pressurized gas from the compressor and supply a gas pressurizing the tank via the fist supply passage.

4. The endoscope washer disinfector according to claim 1,
wherein
the tank consists of a plurality of tanks (11) that contain a plurality of liquid agents for washing and disinfecting the endoscope, every type of agent;
the pressuring member is provided with a signal compressor (22);
the fist supply passage includes a plurality of supply passages each communicating each of the plurality of tanks with the compressor; and
the second supply passage includes a plurality of supply passages each communicating the bath with each of the plurality of tanks.

5. The endoscope washer disinfector according to claim 1,
wherein the detector is a flow sensor (13) disposed in the second supply passage and equipped with a bladed wheel receiving a push from the agent passing the second supply passage and rotating to output the detection signal every rotation.

6. The endoscope washer disinfector according to claim 1,
wherein the discharging member includes
a switch member disposed in the first supply passage and formed to perform a selectable switchover between a path communicating from the pressurizing member to the tank and a further path communicating from the tank to the atmosphere; and
a discharging path connected to the switch member and formed to connect the switch member and the atmosphere.

7. The endoscope washer disinfector according to claim 1, further comprising time memorizing means (S3, 56) that memorizes a period of time starting from a start of the supply of the agent performed by the supply starting means to an end of the supply of the agent performed by the supply ending means.

8. The endoscope washer disinfector according to claim 7, further comprising
detection determining means (57) that determines whether or not the detection signal from the detector stops after the start of the supply of the agent;
time determining means (S10) that determines whether or not the current period of time starting from the start of the supply of the agent reaches the last period of the time memorized by the time memorizing means during the washing and disinfection carried out last time, when it is determined by the detection determining means that the detection signal stops; and
further supply ending means (S11) that ends the supply of the agent to the bath by driving the discharging member to open the inner pressure of the tank to the atmosphere, when it is determined by the time determining means that the current period of time reaches the last period of time.

9. The endoscope washer disinfector according to claim 8, further comprising error processing means (S12) that performs an error process including display of a shortage of the agent in the tank, in cases
where the further supply ending means ends the supply of the agent to the bath.

10. The endoscope washer disinfector according to claim 8,
wherein
the detector is a flow sensor (13) disposed in the second supply passage and equipped with a bladed wheel receiving a push from the agent passing the second supply passage and rotating to output the detection signal every rotation, and
the flow sensor is located at a position in the second supply passage, the position dividing the second supply passage to leave a path portion an inner volume of which is the same as a total amount of the agent necessary for a one-time process including the washing or disinfection, the path portion located nearer to the bath than the flow sensor.

11. The endoscope washer disinfector according to claim 10, further comprising
difference calculating means (S8, S9) that calculates a difference value the newest detection signal and the specified volume, when the detection determining means determines the stop of the detection signal,
output start determining means (S24) that determines whether or not the detector starts to output the detection signal within a period of time corresponding to the period of time memorized by the time memorizing means in the last process,
volume correcting means (S25) that corrects the difference value to another value by adding the difference value to the specified volume, when the output start determining means determines the start of the output of the detection signal,
further determining means (526) that determines whether or not the amount of the agent passing through the second supply passage reaches the corrected specified volume on the basis of the detection signal from the detector, and
further supply ending means (S27) that ends the supply of the agent to the bath by driving the discharging member so that the inside of the tank is opened to the atmosphere, when the further determining means determines that the amount of the agent reaches the corrected specified volume.

12. The endoscope washer disinfector according to claim 10, further comprising
difference calculating means (S8, S9) that calculates a difference value the newest detention information and the specified volume, when the detection determining means determines the stop of the detection signal,
output start determining means (524) that determines whether or not the detector starts to output the detection signal within a period of time corresponding to the period of time memorized by the time memorizing means in the last process, and
still further supply ending means (528) that ends the supply of the agent to the bath by driving the discharging member so that the inside of the tank is opened to the atmosphere, when the output start determining means does not determine the start of the output of the detection signal.

13. The endoscope washer disinfector according to claim 12, further comprising error processing means (529) that performs an error process including display of a shortage of the agent in the tank, in cases
where the still further supply ending means ends the supply of the agent to the bath.

14. A method of supplying a liquid agent from a tank (11) containing a liquid agent for process of an endoscope to a bath (5) in which the endoscope is accommodated and which is for washing and disinfecting the endoscope by pressurizing an inside of the tank, the process including washing, disinfecting, and drying the endoscope, the method comprising steps of:
starting (S1) supply of the agent from the tank to the bath by pressurizing the inside of the tank;
determining (S2, S4) whether or not an amount of the agent being actually supplied to the bath reaches a specified volume on the basis of a detection signal showing the agent pushed outside from the tank in response to the pressurization into the tank; and
ending (S5) the supply of the agent to the bath by making an inner pressure of the tank open to an atmosphere, when it is determined that the amount of the agent reaches the specified volume.

15. The method of claim 14, further comprising steps of:
memorizing (53, S6) a period of time starting from a start of the supply of the agent to an end of the supply of the agent;
determining (S7) whether or not the detection signal stops after the start of the supply of the agent;
determining (S10) whether or not a current period of time starting from the start of the supply of the agent reaches the last period of the time memorized during the washing and disinfection carried out last time, when it is determined that the detection signal stops; and
ending (S11) the supply of the agent to the bath by opening the inner pressure of the tank to the atmosphere, when it is determined that the current period of time reaches the last period of time.

16. The method of claim 15, further comprising a step of performing (S12) an error process including display of a shortage of the agent in the tank, in cases where the supply ending step ends the supply of the agent to the bath.

17. The method of claim 15, further comprising steps of:
calculating (S8, S9) a difference value the newest detection signal and the specified volume, when it is determined that the detection signal stops,
determining (S24) whether or not output of the detection signal starts within a period of time corresponding to the period of time memorized in the last process,
correcting (S25) the difference value to another value by adding the difference value to the specified volume, when it is determined that the output of the detection signal starts,
determining (S26) whether or not the amount of the agent reaches the corrected specified volume on the basis of the detection signal, and
ending (S27) the supply of the agent to the bath by making the inside of the tank open to the atmosphere, when it is determined that the amount of the agent reaches the corrected specified volume.

18. The method of claim 15, further comprising steps of:
calculating (S8, S9) a difference value the newest detection signal and the specified volume, when it is determined that the detection signal stops,
determining (S24) whether or not output of the detection signal starts within a period of time corresponding to the period of time memorized in the last process, and
further ending (S28) the supply of the agent to the bath by making the inside of the tank open to the atmosphere, when it is not determined that the output of the detection signal starts.

19. The method of claim 18, further comprising a step of
performing (S29) an error process including display of a shortage of the agent in the tank, in cases where the further supply ending step ends the supply of the agent to the bath.

20. A unit for supplying liquid agents necessary for a process of an endoscope, to a bath (5) in which the endoscope is accommodated, the process including washing and disinfecting the endoscope, the unit comprising:
a plurality of tanks (11) each containing each of the agents every type of the agent;
a signal pressurizing member (22,19, 17; 22, 19, 39; 22, 17; 22 44) that pressurizes an inside of each of the tanks
a plurality of first supply passages (16) each communicating from each of the tanks to the single pressuring member;
a plurality of second supply passages (12) each communicating from the bath to each of the tanks;
a discharging member (17,28,27; 29, 30; 49, 50) that makes each of the tanks open to an atmosphere;
a plurality of detectors (13) that each detects an amount of the agent extruded from each of the tanks to pass along each of the second supply passages in response to pressurization of the pressurizing member to the tank and outputs a detection signal indicative of the detected amount of the agent; and
control means (40, 13) that controls a selective supply of the agents in the tanks to the bath by pressurizing the inside of each of the tanks via the first supply passages using the pressurizing member, on the basis of each of amounts of the agents corresponding to the detection signal from each of the detectors and a specified volume assigned to each agent.

## Patentansprüche

1. Endoskop-Wäscherdesinfizierer, umfassend:
ein Bad (5), das zum Waschen und Desinfizieren eines darin aufgenommenen Endoskops verwendet wird;
einen Tank (11), der einen flüssigen Wirkstoff speichert, der zum Waschen und Desinfizieren des in dem Bad aufgenommenen Endoskops notwendig ist;
ein Druckelement (22, 19, 17; 22, 19, 39; 22, 17; 22, 44), das ein Inneres des Tanks unter Druck setzt;
einen ersten Zuführdurchlass (16), der den Tank und das Druckelement verbindet;
einen zweiten Zuführdurchlass (12), der das Bad und den Tank verbindet;
ein Ablasselement (17, 28, 27; 29, 30; 49, 50), das einen Innendruck des Tanks an eine Atmosphäre ablässt;
einen Detektor (13), der eine Menge des Wirkstoffs detektiert, der aus dem Tank ausgestoßen wird, um entlang des zweiten Zuführdurchlasses zu strömen, in Antwort auf eine Druckbeaufschlagung von dem Druckelement auf den Tank und ein Detektionssignal ausgibt, das für die detektierte Menge des Wirkstoffs indikativ ist;
ein Zuführstartmittel (S1), das bewirkt, dass der Tank anfängt, dem Bad den Wirkstoff durch den zweiten Zuführdurchlass durch Antreiben des Druckelements zuzuführen, um das Innere des Tanks durch den ersten Zuführdurchlass unter Druck zu setzen;
ein Bestimmungsmittel (S4), das bestimmt, ob die Menge des Wirkstoffs, der durch den zweiten Zuführdurchlass strömt, ein vorgegebenes Volumen erreicht oder nicht, auf der Basis des von dem Detektor ausgegebenen Detektionssignals; und
ein Zuführbeendigungsmittel (S5), das die Zufuhr des Wirkstoffs an das Bad durch Antreiben des Ablasselements beendet, um das Innere des Tanks an die Atmosphäre zu öffnen, wenn das Bestimmungsmittel bestimmt, dass die Menge des Wirkstoff das vorgegebene Volumen erreicht.

2. Endoskop-Wäscherdesinfizierer nach Anspruch 1, wobei das Druckelement mit einem einzigen Kompressor (22) versehen ist, um ein unter Druck gesetztes Gas dem ersten Zuführdurchlass zuzuführen.

3. Endoskop-Wäscherdesinfizierer nach Anspruch 1, wobei das Druckelement mit einem Signalkompressor (22), um ein unter Druck gesetztes Gas bereitzustellen, und einem einzigen Konstantdruck-Tank (19) versehen ist, um einen Innendruck immer auf einem vorgegebenen Wert zu halten, in Übereinstimmung mit dem unter Druck gesetzten Gas von dem Kompressor, und ein Gas bereitzustellen, das den Tank über den ersten Zuführdurchlass unter Druck setzt.

4. Endoskop-Wäscherdesinfizierer nach Anspruch 1, wobei
der Tank aus einer Vielzahl von Tanks (11) besteht, die eine Vielzahl von flüssigen Wirkstoffen jedes Wirkstoff-Typs zum Waschen und Desinfizieren des Endoskops enthalten;
das Druckelement mit einem Signalkompressor (22) versehen ist;
der erste Zuführdurchlass einer Vielzahl von Zuführdurchlässen enthält, von denen jeder jeden der Vielzahl von Tanks mit dem Kompressor in Verbindung setzt; und
der zweite Zuführdurchlass eine Vielzahl von Zuführdurchlässen enthält, von denen jeder das Bad mit jedem der Vielzahl von Tanks in Verbindung setzt.

5. Endoskop-Wäscherdesinfizierer nach Anspruch 1, wobei der Detektor ein Flusssensor (13) ist, der in dem zweiten Zuführdurchlass angeordnet ist und mit einem Laufrad versehen ist, das einen Schwung von dem den zweiten Zuführdurchlass passierenden Wirkstoff aufnimmt und sich dreht, um bei jeder Drehung das Detektionssignal auszugeben.

6. Endoskop-Wäscherdesinfizierer nach Anspruch 1, wobei das Ablasselement enthält
ein Schaltelement, das in dem ersten Zuführdurchlass angeordnet ist und ausgebildet ist, um eine wählbare Umschaltung auszuführen zwischen einem Pfad, der sich von dem Druckelement zu dem Tank erstreckt, und einem weiteren Pfad, der sich von dem Tank an die Atmosphäre erstreckt; und
einen Ablasspfad, der mit dem Schaltelement verbunden ist, und ausgebildet ist, um das Schaltelement und die Atmosphäre zu verbinden.

7. Endoskop-Wäscherdesinfizierer nach Anspruch 1, weiter umfassend ein Zeitspeichermittel (S3, S6), das eine Zeitspanne abspeichert, beginnend von einem Beginn der Zufuhr des Wirkstoffs durchgeführt durch das Zuführstartmittel bis zu einem Ende der Zufuhr des Wirkstoffs durchgeführt durch das Zuführbeendigungsmittel.

8. Endoskop-Wäscherdesinfizierer nach Anspruch 7, weiter umfassend
ein Detektionsbestimmungsmittel (S7), das bestimmt, ob das Detektionssignal von dem Detektor nach dem Beginn der Zufuhr des Wirkstoffs aufhört oder nicht;
ein Zeitbestimmungsmittel (S10), das bestimmt, ob die derzeitige Zeitspanne beginnend von dem Start der Zufuhr des Wirkstoffs die letzte Zeitspanne erreicht oder nicht, die von dem Zeitspeichermittel abgespeichert wurde, während das Waschen und Desinfizieren das letzte Mal ausgeführt wurde, wenn durch das Detektionsbestimmungsmittel ermittelt wird, dass das Detektionssignal aufhört; und
ein weiteres Zuführbeendigungsmittel (S11), das die Zufuhr des Wirkstoffs an das Bad durch Antreiben des Ablasselements beendet, um den Innendruck des Tanks an die Atmosphäre zu öffnen, wenn durch das Zeitbestimmungsmittel bestimmt wird, dass die derzeitige Zeitspanne die letzte Zeitspanne erreicht.

9. Endoskop-Wäscherdesinfizierer nach Anspruch 8, weiter umfassend ein Fehlerverarbeitungsmittel (S12), das einen Fehlerprozess ausführt enthaltend die Anzeige eines Engpasses des Wirkstoffs in dem Tank, in Fällen, in denen das weitere Zuführbeendigungsmittel die Zufuhr des Wirkstoffs an das Bad beendet.

10. Endoskop-Wäscherdesinfizierer nach Anspruch 8, wobei
der Detektor ein Flusssensor (13) ist, der in dem zweiten Zuführdurchlass angeordnet ist und mit einem Laufrad ausgestattet ist, das einen Schwung von dem den zweiten Zuführdurchlass passierenden Wirkstoff aufnimmt und sich dreht, um bei jeder Drehung das Detektionssignal auszugeben, und
der Flusssensor an einer Position in dem zweiten Zuführdurchlass angeordnet ist, wobei die Position den zweiten Zuführdurchlass unterteilt, um einen Pfadabschnitt zu belassen, dessen Innenvolumen das gleiche ist wie eine Gesamtmenge des Wirkstoffs, der für eine einmalige Bearbeitung, enthaltend das Waschen oder Desinfizieren, nötig ist, wobei der Pfadabschnitt näher an dem Bad angeordnet ist als der Flusssensor.

11. Endoskop-Wäscherdesinfizierer nach Anspruch 10, weiter umfassend
ein Differenzberechnungsmittel (S8, S9), das einen Differenzwert berechnet, dem neuesten Detektionssignal und dem vorgegebenen Volumen, wenn das Detektionsbestimmungsmittel die Beendigung des Detektionssignals bestimmt,
ein Ausgabestartbestimmungsmittel (S24), das bestimmt, ob der Detektor beginnt, das Detektionssignal innerhalb einer Zeitspanne auszugeben oder nicht, die der Zeitspanne entspricht, die durch das Zeitspeichermittel bei der letzten Bearbeitung abgespeichert wurde,
ein Volumenkorrekturmittel (S25), das den Differenzwert auf einen anderen Wert korrigiert durch Addieren des Differenzwerts zu dem vorgegebenen Volumen, wenn das Ausgabestartbestimmungsmittel den Beginn der Ausgabe des Detektionssignals bestimmt,
ein weiteres Bestimmungsmittel (S26), das bestimmt, ob die Menge des Wirkstoffs, der durch den zweiten Zuführdurchlass strömt, das korrigierte vorgegebene Volumen erreicht oder nicht, auf der Basis des Detektionssignals von dem Detektor, und
ein weiteres Zuführbeendigungsmittel (S27), das die Zufuhr des Wirkstoffs an das Bad durch Antreiben des Ablasselements beendet, so dass das Innere des Tanks an die Atmosphäre geöffnet wird, wenn das weitere Bestimmungsmittel bestimmt, dass die Menge des Wirkstoffs das korrigierte vorgegebene Volumen erreicht.

12. Endoskop-Wäscherdesinfizierer nach Anspruch 10, weiter umfassend
ein Differenzberechnungsmittel (S8, S9), das einen Differenzwert berechnet, der neuesten Detektionsinformation und dem vorgegebenen Volumen, wenn das Detektionsbestimmungsmittel die Beendigung des Detektionssignals bestimmt,
ein Ausgabestartbestimmungsmittel (S24), das bestimmt, ob der Detektor beginnt, das Detektionssignal innerhalb einer Zeitspanne auszugeben oder nicht, die der Zeitspanne entspricht, die durch das Zeitspeichermittel bei der letzten Bearbeitung abgespeichert wurde, und
noch ein weiteres Zuführbeendigungsmittel (S28), das die Zufuhr des Wirkstoffs an das Bad durch Antreiben des Ablasselements beendet, so dass das Innere des Tanks an die Atmosphäre geöffnet wird, wenn das Ausgabestartbestimmungsmittel nicht den Start der Ausgabe des Detektionssignals bestimmt.

13. Endoskop-Wäscherdesinfizierer nach Anspruch 12, weiter umfassend ein Fehlerverarbeitungsmittel (S29), das einen Fehlerprozess (S25) durchführt enthaltend die Ausgabe eines Engpasses des Wirkstoffs in dem Tank, in Fällen, in denen das noch weitere Zuführbeendigungsmittel die Zufuhr des Wirkstoffs an das Bad beendet.

14. Verfahren zum Zuführen eines flüssigen Wirkstoffs von einem Tank (11), der einen flüssigen Wirkstoff zur Bearbeitung eines Endoskops enthält, an ein Bad (5), in welchem das Endoskop aufgenommen ist und welches zum Waschen und Desinfizieren des Endoskops dient durch unter Druck Setzen eines Inneren des Tanks, wobei die Bearbeitung Waschen, Desinfizieren und Trocknen des Endoskops enthält, wobei das Verfahren die Schritte umfasst:
Beginnen (S1) der Zufuhr des Wirkstoffs von dem Tank an das Bad durch unter Druck Setzen des Inneren des Tanks;
Bestimmen (S2, S4), ob eine Menge des Wirkstoffs, der aktuell dem Bad zugeführt wird, ein vorgegebenes Volumen erreicht oder nicht, auf der Basis eines Detektionssignals, das den Wirkstoff darstellt, der von dem Tank nach außen gedrückt wurde in Antwort auf die Druckbeaufschlagung des Tanks; und
Beenden (S5) der Zufuhr des Wirkstoffs an das Bad, indem ein Innendruck des Tanks gegenüber einer Atmosphäre geöffnet wird, wenn bestimmt wird, dass die Menge des Wirkstoffs das vorgegebene Volumen erreicht.

15. Verfahren nach Anspruch 14, weiter umfassend die Schritte:
Abspeichern (S3, S6) einer Zeitspanne beginnend von einem Beginn der Zufuhr des Wirkstoffs bis zu einem Ende der Zufuhr des Wirkstoffs;
Bestimmten (S7), ob das Detektionssignal nach dem Beginn der Zufuhr des Wirkstoffs aufhört oder nicht;
Bestimmen (S10), ob eine derzeitige Zeitspanne beginnend von dem Beginn der Zufuhr des Wirkstoffs die letzte Zeitspanne erreicht oder nicht, die während des zuletzt ausgeführten Waschens und Desinfizierens abgespeichert wurde, wenn bestimmt wird, dass das Detektionssignal aufhört; und
Beenden (S11) der Zufuhr des Wirkstoffs an das Bad durch Öffnen des Innendrucks des Tanks an die Atmosphäre, wenn bestimmt wird, dass die derzeitige Zeitspanne die letzte Zeitspanne erreicht.

16. Verfahren nach Anspruch 15, weiter umfassend einen Schritt des Ausführens (S12) eines Fehlerprozesses enthaltend die Anzeige eines Engpasses des Wirkstoffs in dem Tank, in Fällen, in denen der Zuführbeendigungsschritt die Zufuhr des Wirkstoffs an das Bad beendet.

17. Verfahren nach Anspruch 15, weiter umfassend die Schritte:
Berechnen (S8, S9) eines Differenzwerts, dem neuesten Detektionssignal und dem vorgegebenen Volumen, wenn ermittelt wird, dass das Detektionssignal aufhört,
Bestimmen (S24), ob die Ausgabe des Detektionssignals innerhalb einer Zeitspanne beginnt oder nicht, die der in der letzten Bearbeitung abgespeicherten Zeitspanne entspricht,
Korrigieren (S25) des Differenzwerts auf einen anderen Wert durch Addieren des Differenzwerts zu dem vorgegebenen Volumen, wenn bestimmt wird, dass die Ausgabe des Detektionssignals beginnt,
Bestimmen (S26), ob die Menge des Wirkstoffs das korrigierte vorgegebene Volumen erreicht oder nicht, auf der Basis des Detektionssignals, und
Beenden (S27) der Zufuhr des Wirkstoffs an das Bad durch Offenlegen des Inneren des Tanks an die Atmosphäre, wenn bestimmt wird, dass die Menge des Wirkstoffs das korrigierte vorgegebene Volumen erreicht.

18. Verfahren nach Anspruch 15, weiter umfassend die Schritte:
Berechnen (S8, S9) eines Differenzwerts, dem neuesten Detektionssignal und dem vorgegebenen Volumen, wenn bestimmt wird, dass das Detektionssignal aufhört,
Bestimmen (S24), ob die Ausgabe des Detektionssignals innerhalb einer Zeitspanne beginnt oder nicht, die der in der letzten Bearbeitung abgespeicherten Zeitspanne entspricht, und
weiteres Beenden (S28) der Zufuhr des Wirkstoffs an das Bad durch Offenlegen des Inneren des Tanks an die Atmosphäre, wenn nicht bestimmt wird, dass die Ausgabe des Detektionssignals beginnt.

19. Verfahren nach Anspruch 18 weiter umfassend einen Schritt des
Ausführens (S29) eines Fehlerprozesses enthaltend die Anzeige eines Engpasses des Wirkstoffs in dem Tank, in Fällen, in denen der weitere Zuführbeendigungsschritt die Zufuhr des Wirkstoffs an das Bad beendet.

20. Einheit zum Zuführen flüssiger Wirkstoffe notwendig für eine Bearbeitung eines Endoskops an ein Bad (5), in welchem das Endoskop aufgenommen ist, wobei die Bearbeitung Waschen und Desinfizieren des Endoskops enthält, wobei die Einheit umfasst:
eine Vielzahl von Tanks (11), von denen jeder jeden der Wirkstoffe jedes Wirkstoff-Typs enthält;
ein Signaldruckelement (22, 19, 17; 22, 19, 39; 22, 17; 22, 44), das ein Inneres jedes der Tanks unter Druck setzt;
eine Vielzahl von ersten Zuführdurchlässen (16), von denen jeder jeden der Tanks mit dem einzigen Druckelement verbindet;
eine Vielzahl von zweiten Zuführdurchlässen (12), von denen jeder von dem Bad mit jedem der Tanks in Verbindung steht;
ein Ablasselement (17, 28, 27; 29, 30; 49, 50), das jeden der Tanks an eine Atmosphäre offenlegt;
eine Vielzahl von Detektoren (13), von denen jeder eine Menge des Wirkstoffs detektiert, der von jedem der Tanks ausgestoßen wurde, um entlang jedem der zweiten Zuführdurchlässe zu strömen, in Antwort auf eine Druckbeaufschlagung des Druckelements an den Tank und ein Detektionssignal ausgibt, das für die detektierte Menge des Wirkstoffs indikativ ist; und
ein Steuermittel (40, 13), das eine gezielte Zufuhr des Wirkstoffs in den Tanks an das Bad durch unter Druck Setzen des Inneren von jedem der Tanks über den ersten Zuführdurchlass steuert, unter Verwendung des Druckelements, auf der Basis von jeder der Mengen der Wirkstoffe, die dem Detektionssignal von jedem der Detektoren und einem vorgegebenen Volumen entsprechen, das jedem Wirkstoff zugewiesen ist.

## Revendications

1. Machine à laver et à désinfecter un endoscope comprenant :
un bain (5) utilisé pour laver et désinfecter un endoscope reçu dans celui-ci ;
un réservoir (11) qui stocke un agent liquide nécessaire pour laver et désinfecter l'endoscope reçu dans le bain ;
un élément de mise sous pression (22, 19, 17 ; 22, 19, 39 ; 22, 17 ; 22, 44) qui met sous pression l'intérieur du réservoir ;
un premier passage d'alimentation (16) qui raccorde le réservoir et l'élément de mise sous pression ;
un deuxième passage d'alimentation (12) qui raccorde le bain et le réservoir ;
un élément de refoulement (17, 28, 27 ; 29, 30 ; 49, 50) qui refoule une pression interne du réservoir vers l'atmosphère ;
un détecteur (13) qui détecte une quantité d'agent extrudé du réservoir pour passer le long du deuxième passage d'alimentation en réponse à la mise sous pression de l'élément de mise sous pression vers le réservoir et qui délivre en sortie un signal de détection indiquant la quantité d'agent détectée ;
un moyen de lancement d'alimentation (S1) qui amène le réservoir à commencer l'alimentation de l'agent vers le bain par l'intermédiaire du deuxième passage d'alimentation en amenant l'élément de mise sous pression à mettre sous pression l'intérieur du réservoir par l'intermédiaire du premier passage d'alimentation ;
un moyen de détermination (S4) qui détermine si oui ou non la quantité d'agent passant à travers le deuxième passage d'alimentation atteint un volume spécifié sur la base du signal de détection délivré en sortie depuis le détecteur ; et
un moyen de fin d'alimentation (S5) qui termine l'alimentation de l'agent vers le bain en amenant l'élément de refoulement à ouvrir l'intérieur du réservoir sur l'atmosphère, lorsque le moyen de détermination détermine que la quantité d'agent atteint le volume spécifié.

2. Machine à laver et à désinfecter un endoscope selon la revendication 1, dans laquelle l'élément de mise sous pression est muni d'un compresseur unique (22) pour délivrer un gaz sous pression vers le premier passage d'alimentation.

3. Machine à laver et à désinfecter un endoscope selon la revendication 1, dans laquelle l'élément de mise sous pression est muni d'un compresseur de signal (22) pour délivrer un gaz sous pression et d'un réservoir à pression constante unique (19) pour toujours maintenir une pression interne à une valeur donnée conformément au gaz sous pression provenant du compresseur et délivrer un gaz mettant sous pression le réservoir via le premier passage d'alimentation.

4. Machine à laver et à désinfecter un endoscope selon la revendication 1, dans laquelle
le réservoir est composé d'une pluralité de réservoirs (11) qui contiennent une pluralité d'agents liquides destinés à laver et à désinfecter l'endoscope, tous types d'agents ;
l'élément de mise sous pression est muni d'un compresseur de signal (22) ;
le premier passage d'alimentation comprend une pluralité de passages d'alimentation chacun mettant en communication chacun de la pluralité de réservoirs avec le compresseur ; et
le deuxième passage d'alimentation comprend une pluralité de passages d'alimentation chacun mettant en communication le bain avec chacun de la pluralité de réservoirs.

5. Machine à laver et à désinfecter un endoscope selon la revendication 1, dans laquelle le détecteur est un capteur de débit (13) disposé dans le deuxième passage d'alimentation et muni d'une roue à aubes recevant une poussée de l'agent traversant le deuxième passage d'alimentation et tournant pour délivrer en sortie le signal de détection à chaque rotation.

6. Machine à laver et à désinfecter un endoscope selon la revendication 1, dans laquelle l'élément de refoulement comprend
un élément de commutation disposé dans le premier passage d'alimentation et formé pour réaliser une commutation pouvant être sélectionnée entre un trajet communicant de l'élément de mise sous pression au réservoir et un autre trajet communicant du réservoir à l'atmosphère ; et
un trajet de refoulement raccordé à l'élément de commutation et formé pour raccorder l'élément de commutation à l'atmosphère.

7. Machine à laver et à désinfecter un endoscope selon la revendication 1, comprenant de plus un moyen de mémorisation de temps (53, S6) qui mémorise une période de temps commençant d'un début d'alimentation en agent réalisée par le moyen de lancement d'alimentation à une fin d'alimentation en agent réalisée par le moyen de fin d'alimentation.

8. Machine à laver et à désinfecter un endoscope selon la revendication 7, comprenant en outre
un moyen de détermination de détection (S7) qui détermine si oui ou non le signal de détection provenant du détecteur s'arrête après le début de l'alimentation en agent ;
un moyen de détermination de temps (S10) qui détermine si oui ou non la période de temps actuelle commençant avec le début de l'alimentation en agent atteint la dernière période de temps mémorisée par le moyen de mémorisation de temps pendant le lavage et la désinfection réalisés la dernière fois, lorsqu'il est déterminé par le moyen de détermination de détection que le signal de détection s'est arrêté ; et
un autre moyen de fin d'alimentation (S11) qui termine l'alimentation en agent vers le bain en amenant l'élément de refoulement à ouvrir la pression interne du réservoir sur l'atmosphère, lorsqu'il est déterminé par le moyen de détermination de temps que la période de temps actuelle atteint la dernière période de temps.

9. Machine à laver et à désinfecter un endoscope selon la revendication 8, comprenant en outre un moyen de traitement d'erreurs (S12) qui réalise un traitement d'erreurs comprenant l'affichage d'un manque en agent dans le réservoir, dans des cas dans lesquels le moyen supplémentaire de fin d'alimentation termine l'alimentation en agent vers le bain.

10. Machine à laver et à désinfecter un endoscope selon la revendication 8, dans laquelle
le détecteur est un capteur de débit (13) disposé dans le deuxième passage d'alimentation et muni d'une roue à aubes recevant une poussée de l'agent passant par le deuxième passage d'alimentation et tournant pour délivrer en sortie le signal de détection à chaque rotation, et
le capteur de débit est placé au niveau d'une position dans le deuxième passage d'alimentation, la position divisant le deuxième passage d'alimentation pour laisser une partie de trajet dont un volume interne est le même que la quantité totale d'agent nécessaire pour un traitement unique comprenant le lavage ou la désinfection, la partie de trajet placée plus près du bain que du capteur de débit.

11. Machine à laver et à désinfecter un endoscope selon la revendication 10, comprenant en outre
un moyen de calcul de différence (S8, S9) qui calcule une valeur de différence entre le nouveau signal de détection et le volume spécifié, lorsque le moyen de détermination de détection détermine l'arrêt du signal de détection,
un moyen de détermination de début de sortie (S24) qui détermine si oui ou non le détecteur commence à délivrer en sortie le signal de détection pendant une période de temps correspondant à la période de temps mémorisée par le moyen de mémorisation de temps lors du dernier traitement,
un moyen de correction de volume (S25) qui corrige la valeur de différence à une autre valeur en ajoutant la valeur de différence au volume spécifié, lorsque le moyen de détermination de début de sortie détermine le début de la sortie du signal de détection,
un autre moyen de détermination (S26) qui détermine si oui ou non la quantité d'agent traversant le deuxième passage d'alimentation atteint le volume spécifié corrigé sur la base du signal de détection provenant du détecteur, et
un autre moyen de fin d'alimentation (S27) qui termine l'alimentation en agent vers le bain en entraînant l'élément de refoulement de sorte que l'intérieur du réservoir est ouvert sur l'atmosphère, lorsque le moyen de détermination supplémentaire détermine que la quantité d'agent atteint le volume spécifié corrigé.

12. Machine à laver et à désinfecter un endoscope selon la revendication 10, comprenant en outre
un moyen de calcul de différence (S8, S9) qui calcule la valeur de différence entre les nouvelles informations de détention et le volume spécifié, lorsque le moyen de détermination de détection détermine l'arrêt du signal de détection,
un moyen de détermination de début de sortie (S24) qui détermine si oui ou non le détecteur commence à délivrer en sortie le signal de détection pendant une période de temps correspondant à la période de temps mémorisée par le moyen de mémorisation de temps lors du dernier traitement, et
encore un autre moyen de fin d'alimentation (S28) qui termine l'alimentation en agent vers le bain en entraînant l'élément de refoulement de sorte que l'intérieur du réservoir est ouvert sur l'atmosphère, lorsque le moyen de détermination de début de sortie ne détermine pas le début de la sortie du signal de détection.

13. Machine à laver et à désinfecter un endoscope selon la revendication 12, comprenant en outre un moyen de traitement d'erreurs (S29) qui réalise un traitement d'erreurs comprenant l'affichage d'un manque en agent dans le réservoir, dans des cas dans lesquels l'autre moyen de fin d'alimentation supplémentaire termine l'alimentation en agent vers le bain.

14. Procédé d'alimentation en agent liquide d'un réservoir (11) contenant un agent liquide pour traitement d'un endoscope à un bain (5) dans lequel l'endoscope est reçu et qui est destiné à laver et à désinfecter l'endoscope en mettant sous pression l'intérieur du réservoir, le traitement comprenant le lavage, la désinfection et le séchage de l'endoscope, le procédé comprenant les étapes consistant à :
lancer (S1) l'alimentation en agent du réservoir au bain en mettant sous pression l'intérieur du réservoir ;
déterminer (S2, S4) si oui ou non une quantité d'agent étant réellement délivrée vers le bain atteint un volume spécifié sur la base d'un signal de détection montrant l'agent poussé à l'extérieur depuis le réservoir en réponse à la mise sous pression dans le réservoir ; et
terminer (S5) l'alimentation en agent vers le bain en amenant une pression interne du réservoir à s'ouvrir vers l'atmosphère, lorsqu'il est déterminé que la quantité d'agent atteint le volume spécifié.

15. Procédé selon la revendication 14, comprenant en outre les étapes consistant à:
mémoriser (S3, S6) une période de temps allant du début de l'alimentation en agent à la fin de l'alimentation en agent ;
déterminer (S7) si oui ou non le signal de détection s'arrête après le début de l'alimentation en agent ;
déterminer (S10) si oui ou non une période de temps actuelle commençant au début de l'alimentation en agent atteint la dernière période de temps mémorisée pendant le lavage et la désinfection réalisés la dernière fois, lorsqu'il est déterminé que le signal de détection s'arrête ; et
terminer (S11) l'alimentation en agent vers le bain en ouvrant la pression interne du réservoir sur l'atmosphère, lorsqu'il est déterminé que la période de temps actuelle atteint la dernière période de temps.

16. Procédé selon la revendication 15, comprenant en outre une étape consistant à réaliser (S12) un traitement d'erreurs comprenant l'affichage d'un manque en agent dans le réservoir, dans des cas dans lesquels l'étape de fin d'alimentation termine l'alimentation en agent vers le bain.

17. Procédé sur la revendication 15, comprenant en outre les étapes consistant à :
calculer (S8, S9) une valeur de différence entre le nouveau signal de détection et le volume spécifié, lorsqu'il est déterminé que le signal de détection s'arrête,
déterminer (S24) si oui ou non la sortie du signal de détection commence pendant une période de temps correspondant à la période de temps mémorisée lors du dernier traitement,
corriger (S25) la valeur de différence à une autre valeur en ajoutant la valeur de différence au volume spécifié, lorsqu'il est déterminé que la sortie du signal de détection commence,
déterminer (S26) si oui ou non la quantité d'agent atteint le volume spécifié corrigé sur la base du signal de détection, et
terminer (S27) l'alimentation en agent vers le bain en amenant l'intérieur du réservoir à s'ouvrir sur l'atmosphère, lorsqu'il est déterminé que la quantité d'agent atteint le volume spécifié corrigé.

18. Procédé selon la revendication 15, comprenant en outre les étapes consistant à:
calculer (S8, S9) une valeur de différence entre le nouveau signal de détection et le volume spécifié, lorsqu'il est déterminé que le signal de détection s'arrête,
déterminer (S24) si oui ou non la sortie du signal de détection commence pendant une période de temps correspondant à la période de temps mémorisée lors du dernier traitement, et
terminer de plus (S28) l'alimentation en agent vers le bain en amenant l'intérieur du réservoir à s'ouvrir sur l'atmosphère, lorsqu'il n'est pas déterminé que la sortie du signal de détection commence.

19. Procédé selon la revendication 18, comprenant en outre une consistant à
réaliser (S29) un traitement d'erreurs comprenant l'affichage d'un manque en agent dans le réservoir, dans des cas dans lesquels l'étape supplémentaire de fin d'alimentation termine l'alimentation en agent vers le bain.

20. Unité d'alimentation en agents liquides nécessaires pour un traitement d'un endoscope vers un bain (5) dans lequel l'endoscope est reçu, le traitement comprenant le lavage et la désinfection de l'endoscope, l'unité comprenant :
une pluralité de réservoirs (11) contenant chacun tous types d'agents ;
un élément de mise sous pression (22, 19, 17 ; 22, 19, 39 ; 22, 17 ; 22, 44) qui met sous pression l'intérieur de chacun des réservoirs
une pluralité de premiers passages d'alimentation (16) communicant chacun de la pluralité de réservoirs à l'élément de mise sous pression unique ;
une pluralité de deuxièmes passages d'alimentation (12) communicant chacun du bain à chacun des réservoirs ;
un élément de refoulement (17, 28, 27 ; 29, 30 ; 49, 50) qui amène chacun des réservoirs à s'ouvrir sur l'atmosphère ;
une pluralité de détecteurs (13) qui détectent chacun une quantité d'agents extrudés de chacun des réservoirs pour passer le long de chacun des deuxièmes passages d'alimentation en réponse à la mise sous pression de l'élément de mise sous pression vers le réservoir et délivrent en sortie un signal de détection indiquant la quantité détectée d'agent ; et
un moyen de commande (40, 13) qui commande une alimentation sélective des agents dans les réservoirs vers le bain en mettant sous pression l'intérieur de chacun des réservoirs via les premiers passages d'alimentation en utilisant l'élément de mise sous pression, sur la base de chacune des quantités d'agents correspondant au signal de détection provenant de chacun des détecteurs et un volume spécifié attribué à chaque agent.
